# EUROPEAN PATENT APPLICATION

(11) **EP 4 706 408 A1**
(43) Date of publication of application: **11.03.2026**
(21) Application number: 24797306.8
(22) Date of filing: 05.04.2024
(51) Int. Cl.: A24B 13/00, A24F 23/02, A61K 31/465, A61K 9/00, A24B 15/16, A24B 15/18, A24B 15/24, A24B 15/28

(54) **METHOD FOR PREPARING POUCH FILLER MATERIAL USING BINDER**

(30) Priority: 24.04.2023 KR 20230053479
(71) Applicant: KT&G Corporation, Daedeok-gu Daejeon 34337 (KR)
(72) Inventor: CHA, Sung Je, Daejeon 34128 (KR); KI, Sung Jong, Daejeon 34128 (KR); JEOUNG, Eunmi, Daejeon 34128 (KR); SHIN, Jun Won, Daejeon 34128 (KR)
(74) Representative: Ter Meer Steinmeister & Partner
(86) International application number: PCT/KR2024/004490
(87) International publication number: WO 2024/225649

(57) **Abstract**

The present invention provides a method for preparing a pouch filler material, comprising the steps of: (S1) preparing a solid filler material including a binder; (S2) preparing a particle size control solution including a processing aid; and (S3) spraying the particle size control solution onto the solid filler material while stirring the solid filler material.

## Description

This application claims the benefit of Korean Patent Application No. 10-2023-0053479 filed on April 24, 2023, in the Korean Intellectual Property Office, the entire disclosure of which is incorporated herein by reference for all purposes.

### BACKGROUND

### 1. Field of the Invention

One or more embodiments relate to a method of preparing a pouch filling material using a binder.

### 2. Description of the Related Art

A method of preparing a filler which is an internal filling material in the form of a pouch, such as snus, is generally performed through a simple preparation process including sorting constituent materials in the form of a powder by particle sizes, granulizing the material while stirring, drying the materials, and sorting the materials by particle sizes. The characteristics such as a particle size, distribution, and the like of the filler are determined by the granulizing.

In a technology of the related art, in such granulizing, a granulizing solution is prepared by dissolving various binders in a processing aid, and put into another solid-phase powder constituent material being stirred, and dried to prepare a filler having a characteristic of a certain particle size.

At this time, a content of binder is adjusted to adjust the characteristic of the particle size such as the size, distribution, and the like. However, as the content of binder in the granulizing solution increases, a viscosity of the granulizing solution increases to reduce manufacturing workability. When a content of the processing aid in the granulizing solution is increased to lower the viscosity, lumping may occur during the process of preparing the filler, which may degrade manufacturing workability.

### SUMMARY

An object of the present disclosure is to adjust a particle size characteristic of a filling material by spraying a particle size control solution including a processing aid during stirring a material in a powder form including a binder.

However, goals to be achieved are not limited to those described above, and other goals not mentioned above are clearly understood by one of ordinary skill in the art from the following description.

According to an aspect, there is provided method for preparing a pouch filling material including step S1 of preparing a solid-phase filling material including a binder, step S2 of preparing a particle size control solution including a processing aid, and step S3 of spraying the particle size control solution onto the solid-phase filling material while stirring the solid-phase filling material.

The method may further include step S4 of performing granulizing by mixing the particle size control solution with the solid-phase filling material after step S3.

The method may further include step S5 of performing drying at a temperature of room temperature to 80°C for 2 to 5 hours after step S4.

The method may further include one or more steps of step a of sieving a raw material of the solid-phase filling material before step S1, step b of sieving the mixed filling material after step S4, and step c of sieving the dried material after step S5.

The binder may be included in an amount of 20% by weight or less with respect to a total weight of the solid-phase filling material, and the particle size control solution may be included in an amount of 5% by weight to 30% by weight with respect to the total weight of the solid-phase filling material.

The binder may include one or more selected from a group consisting of hydroxypropyl cellulose (HPC), hydroxypropyl methylcellulose (HPMC), polyvinylpyrrolidone (PVP), sodium alginate, xanthan gum, guar gum, gellan gum, and gum arabic.

The solid-phase filling material may include one or more of an activating material, a bulking agent, a pH control agent, a flavoring material, and a sweetener.

The activating material may include one or more activating materials selected from a group consisting of nicotine, vitamin, caffeine, a salt thereof, and a derivative thereof.

The bulking agent may include one or more of one or more cellulose selected from a group consisting of microcrystalline cellulose (MCC), methylcellulose, hydroxypropyl cellulose, hydroxypropyl methyl cellulose, hydroxyethyl cellulose, and carboxymethyl cellulose, one or more sugar alcohols selected from a group consisting of isomalt, erythritol, sorbitol, arabitol, ribitol, maltitol, dulcitol, iditol, mannitol, xylitol, and lactitol, and non-tobacco plant material.

The pH control agent may include one or more selected from a group consisting of sodium hydroxide, potassium hydroxide, sodium carbonate, potassium carbonate, sodium bicarbonate, and sodium sesquicarbonate (Na₃H(CO₃)₂).

The flavoring material may include one or more selected from a group consisting of vanilla, coffee, chocolate/cocoa, cream, mint, spearmint, menthol, peppermint, wintergreen, eucalyptus, lavender, cardamom, nutmeg, cinnamon, clove, cascarilla, Santalum album, honey, ginger, anise, sage, licorice, lemon, orange, apple, peach, lime, cherry, and strawberry.

The sweetener may include one or more selected from a group consisting of fructose, sucrose, glucose, maltose, mannose, galactose, lactose, stevia, honey, sucralose, isomaltose, maltodextrin, saccharin, and aspartame.

The processing aid may include water or alcohol.

Additional aspects of example embodiments will be set forth in part in the description which follows and, in part, will be apparent from the description, or may be learned by practice of the disclosure.

In a method of preparing a pouch filling material according to an aspect of the present disclosure, a viscosity of a particle size control solution used is not high and lumps are not generated. Therefore, the method is advantageous that manufacturing workability is excellent, the characteristic of the particle size of the filling material may easily adjusted, and the characteristic of a uniform particle size may be obtained.

### BRIEF DESCRIPTION OF THE DRAWINGS

These and/or other aspects, features, and advantages of the invention will become apparent and more readily appreciated from the following description of example embodiments, taken in conjunction with the accompanying drawing of which:

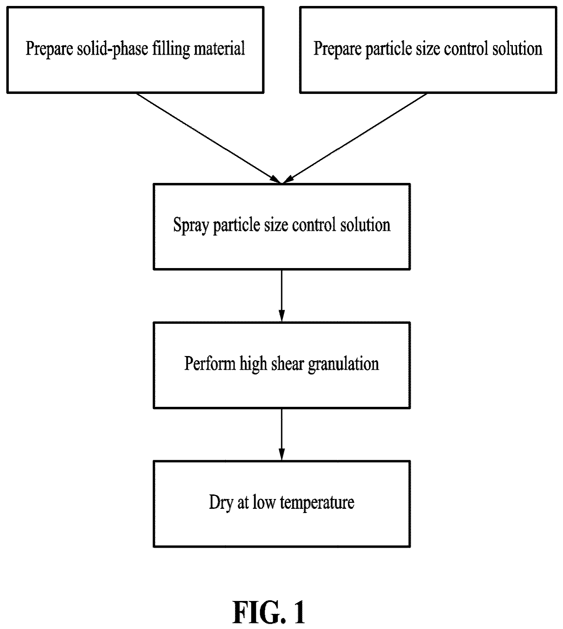

FIG. 1 is a drawing illustrating a schematic flowchart of a method of preparing a pouch filling material according to an embodiment of the present disclosure.

### DETAILED DESCRIPTION

Hereinafter, embodiments will be described in detail with reference to the accompanying drawings. However, various alterations and modifications may be made to the embodiments. Here, the embodiments are not construed as limited to the disclosure. The embodiments should be understood to include all changes, equivalents, and replacements within the idea and the technical scope of the disclosure.

The terminology used herein is for the purpose of describing particular embodiments only and is not to be limiting of the embodiments. The singular forms "a", "an", and "the" are intended to include the plural forms as well, unless the context clearly indicates otherwise. It will be further understood that the terms "comprises/comprising" and/or "includes/including" when used herein, specify the presence of stated features, integers, steps, operations, elements, and/or components, but do not preclude the presence or addition of one or more other features, integers, steps, operations, elements, components and/or groups thereof.

Unless otherwise defined, all terms including technical and scientific terms used herein have the same meaning as commonly understood by one of ordinary skill in the art to which the embodiments belong. It will be further understood that terms, such as those defined in commonly-used dictionaries, should be interpreted as having a meaning that is consistent with their meaning in the context of the relevant art and will not be interpreted in an idealized or overly formal sense unless expressly so defined herein.

When describing the embodiments with reference to the accompanying drawings, like reference numerals refer to like components and a repeated description related thereto will be omitted. In the description of embodiments, detailed description of well-known related structures or functions will be omitted when it is deemed that such description will cause ambiguous interpretation of the present disclosure.

In addition, the terms first, second, A, B, (a), and (b) may be used to describe constituent elements of the embodiments. These terms are used only for the purpose of discriminating one component from another component, and the nature, the sequences, or the orders of the components are not limited by the terms.

A component, which has the same common function as a component included in any one embodiment, will be described by using the same name in other embodiments. Unless otherwise mentioned, the descriptions of an embodiment may be applicable to other embodiments and thus, repeated descriptions will be omitted for conciseness.

According to an embodiment of the present disclosure, there is provided a method of preparing a pouch filling material including:
step S1 of preparing a solid-phase filling material including a binder;
step S2 of preparing a particle size control solution including a processing aid; and
step S3 of spraying the particle size control solution onto the solid-phase filling material while stirring the solid-phase filling material.

In order to prepare the pouch filling material, first, the solid-phase filling material and the particle size control solution are prepared. At this time, the order of preparation of the solid-phase filling material and the particle size control solution is not limited. In other words, the particle size control solution may be prepared after preparing the solid-phase filling material, the solid-phase filling material may be prepared after preparing the particle size control solution, or these may be prepared at the same time.

However, the binder used in the preparing of the pouch filling material is prepared to be included in the solid-phase filling material, rather than the particle size control solution, and a raw material of the solid-phase filling material may be primarily sorted by particle sizes through sieving. Since the raw material of the solid-phase filling material may be lumpy when mixing due to its characteristic, uniformity of mixing may be affected when the raw material is mixed as it is to prepare the solid-phase filling material. Accordingly, it is preferable to prevent lumpiness through sieving.

The solid-phase filling material may include an activating material, a bulking agent, a pH control agent, a flavoring material, a sweetener, or the like, in addition to the binder, and the binder may include hydroxypropyl cellulose (HPC), hydroxypropyl methylcellulose (HPMC), polyvinylpyrrolidone (PVP), sodium alginate, xanthan gum, guar gum, gellan gum, gum arabic, or the like.

The activating material is a material that is absorbed through the oral mucosa or gums, specifically, may include nicotine, vitamin, caffeine, amino acid, a salt or derivative thereof, and may desirably include nicotine. The nicotine may be synthetic nicotine and/or a nicotine extract from tobacco leaves.

The bulking agent may include cellulose, sugar alcohol, a non-tobacco plant material, or the like. The cellulose may include microcrystalline cellulose (MCC), cellulose, a cellulose derivative, or the like, and specific examples thereof may include MCC, methylcellulose, hydroxypropyl cellulose, hydroxypropyl methyl cellulose, hydroxyethyl cellulose, carboxymethyl cellulose, and the like. In addition, specific examples of the sugar alcohol may include isomalt, erythritol, sorbitol, arabitol, ribitol, maltitol, dulcitol, iditol, mannitol, xylitol, lactitol, and the like. Furthermore, examples of the non-tobacco plant material are not particularly limited, and may include a coconut shell, vegetable fiber, tea, herbs, coffee, fruits, and the like.

In addition, specific types of the pH control agent that may be used according to an embodiment of the present disclosure may include metal hydroxide such as sodium hydroxide and potassium hydroxide, metal carbonate such as sodium carbonate and potassium carbonate, sodium bicarbonate, sodium sesquicarbonate (Na₃H(CO₃)₂), and the like.

The flavoring material used according to an embodiment of the present disclosure is also referred to as a "flavoring agent", and refers to any aromatic material capable of changing a sensing characteristic related to an oral product. The flavoring material may be a natural flavoring material or a synthetic flavoring material, and specific types thereof may include vanilla, coffee, chocolate/cocoa, cream, mint, spearmint, menthol, peppermint, wintergreen, eucalyptus, lavender, cardamom, nutmeg, cinnamon, clove, cascarilla, Santalum album, honey, ginger, anise, sage, licorice, lemon, orange, apple, peach, lime, cherry, strawberry, and the like, but are not limited thereto.

The flavoring material is described above, for example, as being included in the solid-phase filling material in the method of preparing the pouch filling material of the present disclosure, however, the flavoring material may be included in the particle size control solution or in both the solid-phase filling material and the particle size control solution.

Meanwhile, in a case of the sweetener, a natural sweetener or an artificial sweetener may be used, and specific types thereof may include fructose, sucrose, glucose, maltose, mannose, galactose, lactose, stevia, honey, sucralose, isomaltose, maltodextrin, saccharin, aspartame, and the like, but are not limited thereto.

When the binder is included in the solid-phase filling material, an appropriate content of the binder may be 20% by weight or less, and desirably 10% by weight to 14% by weight with respect to a total weight of the solid-phase filling material. When the content of the binder exceeds 20% by weight, a particle size of granules increases to cause lumping during the preparation, which may degrade manufacturing workability or make the preparing operation impossible. When the content of the binder is less than 10% by weight, the binding of the solid-phase filling material may not be performed in an excellent manner, and thus, a large proportion of fine particles (to 100 µm) may be obtained.

Meanwhile, when preparing the particle size control solution, a processing aid such as water or alcohol may be used as a solvent. In this case, the particle size control solution may include the flavoring agent described above in detail and/or a liquid material such as a moisturizing agent, for example, propylene glycol or glycerin. The moisturizing agent may not only impart moist quality to a filler, but also give an antiseptic effect.

When the preparation of the particle size control solution and the solid-phase filling material is completed, the particle size control solution may be sprayed onto the solid-phase filling material while stirring the solid-phase filling material (S3), and then, granulizing is performed by mixing the particle size control solution with the solid-phase filling material through high-speed mixing (S4). An appropriate content of the particle size control solution used herein may be 5% by weight to 30% by weight with respect to the total weight of the solid-phase filling material, and specifically, the appropriate content of the particle size control solution may partially vary depending on the range of the appropriate content of the binder, and may be desirably 10% by weight to 20% by weight. For example, when a HPC binder is used in an amount of 10% by weight, the appropriate content of the particle size control solution may be more desirably 12% by weight to 16% by weight with respect to the total weight of the solid-phase filling material.

Meanwhile, when the content of the particle size control solution is less than 5% by weight, the effect of binding is reduced, which may cause the presence of fine particles of the solid-phase filling material. When the content of the particle size control solution exceeds 30% by weight, lumping occurs during preparation, which may degrade manufacturing workability or make the preparation operation impossible.

The granulizing may be performed by a dry or wet granulizing method, however, herein, the granulizing may be performed particularly as batch-type high shear granulation (wet granulation using a bottom-driven type vertical high-shear granulator) which is generally known in the related industry.

After the granulizing, the mixed solution is dried at a low temperature of room temperature to 80°C for 2 to 5 hours (S5) to volatilize a solvent, thereby finally preparing a filling material in a powdered solid state.

In addition, optionally, before and after performing the low-temperature drying, the mixed filling material may be sieved or the dried powder may be sieved to finally obtain a pouch filling material having a desired uniform particle size.

Hereinafter, the configuration of the present disclosure and effects thereof will be described in more detail through examples and comparative examples. However, the examples are merely intended for the purpose of describing the disclosure in more detail, and thus, the scope of the disclosure is not limited to the examples.

### -Example: Preparation of pouch filling material

### 1) Example 1

A solid-phase filling material containing 10% by weight of hydroxypropyl cellulose (HPC), which is a binder, was prepared, and 2% by weight of menthol and 15% by weight of alcohol with respect to a total weight of the solid-phase filling material were mixed to prepare a particle size control solution.

While stirring the prepared solid-phase filling material, the particle size control solution was sprayed onto the solid-phase filling material.

Then, the granulizing was carried out by high speed mixing, and the resultant material was dried at a low temperature (50°C) for 3 hours and sieved through 40 mesh (425 µm) to prepare a final pouch filling material.

### 2) Example 2

A final pouch filling material was prepared in the same manner as in Example 1 except that the solid-phase filling material was prepared to contain 2% by weight of HPC which is a binder.

### 3) Example 3

A final pouch filling material was prepared in the same manner as in Example 1 except that the solid-phase filling material was prepared to contain 16% by weight of HPC which is a binder.

### 4) Comparative Example 1

10% by weight of HPC, which is a binder, and 2% by weight of menthol, which is a flavoring agent, were mixed with 15% by weight of alcohol, which is a processing aid to prepare a particle size control solution, and a solid-phase filling material not containing the binder was prepared.

A final pouch filling material was prepared in the same manner as in Example 1 except that the particle size control solution and the solid-phase filling material were prepared as described above.

### 5) Comparative Example 2

10% by weight of HPC, which is a binder, and 2% by weight of menthol, which is a flavoring agent, were mixed with 30% by weight of alcohol, which is a processing aid to prepare a particle size control solution, and a solid-phase filling material not containing the binder was prepared.

A final pouch filling material was prepared in the same manner as in Example 1 except that the particle size control solution and the solid-phase filling material were prepared as described above.

In a case of Comparative Example 1, it is difficult to evenly spray the particle size control solution onto the solid-phase filling material being stirred due to a high viscosity of the particle size control solution that is mixed with the binder, and the particle size control may not be easily performed, resulting in non-uniform particle sizes of the filling material.

In a case of Comparative Example 2, the viscosity of the particle size control solution was lower than that in Comparative Example 1, however, lumping has occurred (average particle size of approximately 5 to 10 mm) during high speed mixing of the solid-phase filling material, onto which the particle size control solution was sprayed, due to an excessive amount of the particle size control solution.

In contrast, in a case of Example 1, the workability was excellent during preparing a pouch filling material, and a characteristic of an average particle size (average particle size: 253 µm, a proportion of fine particles of less than 100 µm: approximately 1.2%) of the prepared filling material was uniform.

Meanwhile, in a case of Examples 2 and 3, the manufacturing workability was excellent without lumping of the pouch filling material compared to Comparative Examples 1 and 2. However, compared to Example 1, in Example 2, the proportion of fine particles having an average particle size of less than 100 µm was high as approximately 23%, and in Example 3, the overall particle size was high and a yield of a pouch filling material subjected to sieving for a desired particle size was approximately 17%.

While the embodiments are described with reference to drawings, it will be apparent to one of ordinary skill in the art that various alterations and modifications in form and details may be made in these embodiments without departing from the spirit and scope of the claims and their equivalents. For example, suitable results may be achieved if the described techniques are performed in a different order, and/or if components in a described system, architecture, device, or circuit are combined in a different manner, and/or replaced or supplemented by other components or their equivalents.

Therefore, other implementations, other embodiments, and equivalents to the claims are also within the scope of the following claims.

## Claims

1. A method for preparing a pouch filling material comprising:
step S1 of preparing a solid-phase filling material comprising a binder;
step S2 of preparing a particle size control solution comprising a processing aid; and
step S3 of spraying the particle size control solution onto the solid-phase filling material while stirring the solid-phase filling material.

2. The method of claim 1, further comprising:
step S4 of performing granulizing by mixing the particle size control solution with the solid-phase filling material after step S3.

3. The method of claim 2, further comprising:
step S5 of performing drying at a temperature of room temperature to 80°C for 2 to 5 hours after step S4.

4. The method of claim 3, further comprising one or more steps of:
step a of sieving a raw material of the solid-phase filling material before step S1;
step b of sieving the mixed filling material after step S4; and
step c of sieving the dried material after step S5.

5. The method of claim 1, wherein
the binder is included in an amount of 20% by weight or less with respect to a total weight of the solid-phase filling material, and
the particle size control solution is included in an amount of 5% by weight to 30% by weight with respect to the total weight of the solid-phase filling material.

6. The method of claim 1, wherein the binder comprises one or more selected from a group consisting of hydroxypropyl cellulose (HPC), hydroxypropyl methylcellulose (HPMC), polyvinylpyrrolidone (PVP), sodium alginate, xanthan gum, guar gum, gellan gum, and gum arabic.

7. The method of claim 1, wherein the solid-phase filling material comprises one or more of an activating material, a bulking agent, a pH control agent, a flavoring material, and a sweetener.

8. The method of claim 7, wherein the activating material comprises one or more activating materials selected from a group consisting of nicotine, vitamin, caffeine, a salt thereof, and a derivative thereof.

9. The method of claim 7, wherein the bulking agent comprises one or more of one or more cellulose selected from a group consisting of microcrystalline cellulose (MCC), methylcellulose, hydroxypropyl cellulose, hydroxypropyl methyl cellulose, hydroxyethyl cellulose, and carboxymethyl cellulose, one or more sugar alcohols selected from a group consisting of isomalt, erythritol, sorbitol, arabitol, ribitol, maltitol, dulcitol, iditol, mannitol, xylitol, and lactitol, and non-tobacco plant material.

10. The method of claim 7, wherein the pH control agent comprises one or more selected from a group consisting of sodium hydroxide, potassium hydroxide, sodium carbonate, potassium carbonate, sodium bicarbonate, and sodium sesquicarbonate (Na₃H(CO₃)₂).

11. The method of claim 7, wherein the flavoring material comprises one or more selected from a group consisting of vanilla, coffee, chocolate/cocoa, cream, mint, spearmint, menthol, peppermint, wintergreen, eucalyptus, lavender, cardamom, nutmeg, cinnamon, clove, cascarilla, Santalum album, honey, ginger, anise, sage, licorice, lemon, orange, apple, peach, lime, cherry, and strawberry.

12. The method of claim 7, wherein the sweetener comprises one or more selected from a group consisting of fructose, sucrose, glucose, maltose, mannose, galactose, lactose, stevia, honey, sucralose, isomaltose, maltodextrin, saccharin, and aspartame.

13. The method of claim 1, wherein the processing aid comprises water or alcohol.
